# EUROPEAN PATENT SPECIFICATION

(11) **EP 1 693 014 B1**
(45) Date of publication and mention of the grant of the patent: **21.01.2009**
(21) Application number: 06250806.4
(22) Date of filing: 15.02.2006
(51) Int. Cl.: A61B 18/00, A61B 18/04, H05H 1/00, H01J 37/244, H01J 37/32

(54) **Apparatus and method for calibration of thermal energy delivery in plasma tissue surface treatment system**
Vorrichtung und Verfahren zur Kalibrierung der Abgabe von thermischer Energie bei einem Plasmagerät zur Behandlung von Gewebeoberflächen
Appareil et procédé permettant la calibration de la libération d'energie thermique dans un dispositif de traitement de surface des tissus avec plasma

(30) Priority: 17.02.2005 GB 0503380
(43) Date of publication of application: 23.08.2006
(73) Proprietor: RHYTEC LIMITED, Hungerford, Berkshire RG17 0UN (GB)
(72) Inventor: Penny, Keith, Wellesley, MA 02482 (US); Goble, Nigel Mark, Newbury Berkshire RG20 0HX (GB); Goble, Colin Charles Owen, Oxfordshire RG9 2LA (GB)
(74) Representative: Blatchford, William Michael

(56) References cited:
- WO-A-00/32127
- US-A1- 2002 161 362
- US-A1- 2005 149 012
- US-B1- 6 475 215
- US-B1- 6 920 312
- PELAH I ET AL: "Differential calorimeter for measurement of absorbed energy in laser-produced plasmas" REVIEW OF SCIENTIFIC INSTRUMENTS USA, vol. 48, no. 8, August 1977 (1977-08), pages 1068-1071, XP002377334 ISSN: 0034-6748
- KULIK P P ET AL: "METHOD FOR MEASUREMENT OF THERMAL-FLUX DISTRIBUTION IN LOW-TEMPERATURE PLASMA" INSTRUMENTS AND EXPERIMENTAL TECHNIQUES, CONSULTANTS BUREAU. NEW YORK, US, vol. 31, no. 2, PART 2, 1 March 1988 (1988-03-01), pages 410-412, XP000004038 ISSN: 0020-4412

## Description

This invention relates to a tissue treatment system including a radio frequency (r.f.) generator and a treatment instrument connectible to the generator and to a source of ionisable gas for producing a plasma jet. The primary use of the system is skin resurfacing. Also disclosed is a method of regenerating the reticular architecture of the dermis.

A tissue treatment system is disclosed in U.S. Patents Nos. 6,723,091 issued 20 April 2004, and 6,629,974 issued 7 October 2003 and U.S. Patent Application Serial No. 10/727,765 filed March 5, 2004.

In this known system, a handheld treatment instrument has a gas conduit terminating in a plasma exit nozzle. There is an electrode associated with the conduit, and this electrode is coupled to a separate r.f. power generator which is arranged to deliver r.f. power to the electrode for creating a plasma from gas fed through the conduit. The delivered radio frequency power is typically at UHF frequencies in the region of 2.45GHz and the instrument includes a structure resonant in that frequency region in order to provide an electric field concentration in the conduit for striking the plasma upstream of the exit nozzle, the plasma forming a jet which emerges from the nozzle and which can be used to effect local heating of a tissue surface.

The clinical effect of a system that delivers pulsed energy to the tissue of a patient is dependent on the amount of energy delivered, more particularly the instantaneous power integrated over the time of activation. It is, therefore, important to be able to confirm that the energy delivered by the system corresponds to the setting of the generator (which may be set by the user) and is within the specification of the system.

Typically, the power generated is monitored at one or more points and is compared with an expected value or range of values. In a closed-loop control system, feedback is used to adjust the generation of power. In an open-loop control system, deviation of one or more electrical parameters associated with the generator output from respective expected values by more than a certain degree may result in an error or fault indication, or in halt of the treatment by a control system forming part of the generator.

The above mentioned U.S. Patent No. 6,629,974 discloses a method of skin treatment using a plasma jet, as described above. The invention disclosed in this document is characterised by the energy of pulses of radio frequency power used to create the plasma being such that a small copper disc placed at a predetermined spacing from the nozzle of the instrument is heated to increase its temperature by a predetermined value. This so-called "copper disc test" is defined in order that the disclosed instrument can be compared to other devices.

WO00/32127 discloses a system for the electrosurgical treatment of vascular disorders using an instrument having one or more temperature sensors at or near the distal end of either or both of a pair of exposed electrodes which, in use, are buried in the tissue to be treated. These sensors provide feedback signals during treatment to allow maintenance of the temperature in the tissue within a predetermined range.

In Pelah, I. et al "Differential calorimeter for measurement of absorbed energy in laser-produced plasmas" Review of Scientific Instruments USA, vol. 48, no. 8, August 1997 (1977-08), pages 1068-1071, there is disclosed a differential calorimeter for measuring optical energy absorbed by a target in connection with laser-induced fusion experiments.

In Kulik, P.P. et al "Method for Measurement of Thermal-Flux Distribution in Low-Temperature Plasma". Instruments and Experimental Techniques, Consultants Bureau. New York, US, vol. 31, no. 2, Part 2, 1 March 1988 (1988-03-01), pages 410-412, jet of nitrogen plasma from an electric-arc plasmatron with a power of 30kW is directed onto a sensor having an array of thermocouples. The sensor is moved through the plasma cross-section during measurement.

It is an object of the invention to provide an improved means of controlling treatment energy in a tissue treatment system.

According to a first aspect of this invention, a tissue treatment system includes a radio frequency (r.f.) generator, a treatment instrument connectible to the generator and to a source of ionisable gas and operable to produce a plasma jet at a nozzle of the instrument when supplied with the ionisable gas and energised by the generator, and a calibration device which comprises: a target element having a target surface area, a transducer arranged to produce an electrical signal indicative of a temperature change of the target element within the target surface area, and a locating feature for locating the nozzle of the instrument in juxtaposition with the target surface area whereby, in operation of the instrument when in the receptacle, the plasma jet is incident upon the target surface area to cause the transducer to generate a calibration signal for adjusting the energy output of the generator, and an output connected to the generator for coupling the calibration signal to the generator, the generator including an energy output adjuster for adjusting the r.f. energy output of the generator, the adjuster being configured to adjust the r.f. energy output in response to the calibration signal.

In this way, the delivery of energy is calibrated with respect to delivery at the point of application so that the collective variation of components and processes in the generator, power supplies, and in transmission and connection paths is taken into account. Looked at it in a different way, the energy is calibrated in terms of the energy delivered at or beyond the distal end of a cable connecting the treatment instrument with the r.f. generator. Although the r.f. power produced by the generator may be monitored within the generator itself, it is possible, using means described above, to allow for imperfections in the connecting cable where significant power may be lost through heating. Cable degradation can occur through ageing or through other faults that introduce losses.

Even if r.f. power were to be measured at the distal end of the cable, calibrating energy delivered at the point of application allows adjustment to correct for, e.g., timing drift from the gas supply, gas supply leaks, degradation of an electrode within the treatment instrument, and degradation of the instrument nozzle or damage to the nozzle as a result of bad storage or handling.

In an alternative aspect, the invention may be seen as a means of calibrating the energy delivered to a simulated target by a gas plasma.

In a further aspect, means are provided for determining the energy delivered by a pulsed gas plasma using a measuring device, for instance a calorimeter which is integral to the plasma generating system and which may be electrically or otherwise connected to the generator so that the generator may be used to determine, indicate and/or control the plasma generation process.

The preferred system is a plasma skin resurfacing system which controls the damage and modification of superficial layers of a patient's skin. Such a clinical effect is achieved by rapidly heating the outer layers of the skin from the application of energy stored by ionising nitrogen gas. The plasma emerges from the nozzle of the treatment instrument which is typically held a few millimetres above the tissue surface to be treated.

The energy output adjuster may form part of a controller within the generator which is configured to perform an automatic calibration process. This process may comprise operating the generator to feed to the instrument a predetermined r.f. energy waveform, such as a series of bursts of r.f. energy lasting for a predetermined duration, to cause generation of a calibration plasma jet. This jet is directed at the target surface area of the calibration device, the calibration signal generated as a result of the calibration jet heating the target element being processed to obtain a calibration value which is compared with a reference value, so that the adjuster may be operated automatically in response to any deviation of the calibration value from the reference value by more than a predetermined degree in order to set the r.f. energy output for subsequent operation of the system in treating a patient's tissue. The calibration value is preferably a function of the combination of the levels of successive signal pulses which are generated by the transducer and which correspond to the plasma pulses.

Advantageously, the calibration device is in the form of a calorimeter incorporating a target element such as a metallic plate, a body portion underlying the metallic plate, and a temperature difference sensor, e.g., a Peltier device, located between the metallic plate and the calorimeter body portion. The sensor is arranged to produce an electrical output signal indicative of the difference in temperature between the metallic plate and the body portion.

In the preferred embodiment, the generator is contained within a floor-standing generator housing and the treatment instrument is coupled to the generator housing by a cable for conveying r.f. energy from the generator to the instrument. The calibration device may be mounted in or on the generator housing and has an associated instrument holder which can be used to hold the treatment instrument when not in use and, more particularly, receives the instrument for calibration, the instrument holder being shaped to locate the instrument longitudinally and transversely with respect to the target element. Indeed, the instrument and the instrument holder may have a corresponding asymmetrical cross-section in order that the rotational position of the instrument with respect to the calibration device is also defined.

According to another aspect of the invention, a method of calibrating a tissue treatment system comprises: placing the treatment instrument in a pre-defined location with respect to the target element; causing gas to be supplied to the instrument and simultaneously causing a burst of r.f. energy to be delivered to the instrument with a predetermined waveform and duration so as to generate a plasma jet exiting the nozzle and incident upon the target element; automatically evaluating a calibration signal generated by the calibration device at the said output and representative of an output of the transducer resulting from heating of the target element by the plasma jet; and setting the r.f. energy output level of the generator according to the evaluation of the calibration signal.

Human skin has two principal layers: the epidermis, which is the outer layer and typically has a thickness of around 120µ in the region of the face, and the dermis which is typically 20-30 times thicker than the epidermis, and contains hair follicles, sebaceous glands, nerve endings and fine blood capillaries. By volume the dermis is made up predominantly of the protein collagen.

Ageing and exposure to ultraviolet (UV) light result in changes to the structure of the skin, these changes including a loss of elasticity, sagging, wrinkling and a pallor or yellowing of the skin consistent with reduced vascularity. The background to these effects is explained in our co-pending patent application entitled "Method of Regenerating the Reticular Architecture of the Dermis" filed on even date herewith.

The device of claim 1 has use in a cosmetic method of regenerating the reticular architecture of tissue using a tissue treatment system having a handheld tissue treatment instrument which emits a thermal energy beam, wherein the method comprises calibrating the energy delivery of the system by placing the treatment instrument in registry with a temperature-sensing target device, operating the system to direct a thermal energy beam at the target device, adjusting at least one energy setting of the system in response to an output of the target device to calibrate the system for energy output, moving the treatment instrument to a treatment position over an area of tissue to be treated, and operating the system to cause thermal energy to be delivered to the tissue surface using the calibrated system.

The device of claim 1 also has use in a cosmetic method of regenerating the reticular architecture of tissue using a source of thermal energy comprising the steps of: calibrating the source by operating the source in conjunction with a delivered- energy-sensing target device and adjusting the source in response to an output of the device, and operating the adjusted source to form first and second adjacent regions of thermally modified tissue in the region of the DE junction, said first region overlying said second region and being thermally modified to a greater extent than said second region.

The device of claim 1 has use in a cosmetic method of regenerating the reticular architecture of the dermis using a source of thermal energy comprises the steps of: calibrating the source by operating the source in conjunction with a delivered-energy-sensing target device and adjusting the source in response to an output of the device, and operating the adjusted source and directing it at the surface of the skin to form first and second adjacent regions of thermally-modified tissue in the region of the epidermis and dermis of the skin, said first region overlying said second region and being thermally modified to an extent that it separates from said second region some days after the delivery of the thermal energy, and the depth of said separation being dependent on the amount of energy delivered and the thermal capacity of the skin.

Preferably, the thermal energy source is operated for a single pass over the skin surface, the thermal energy source being arranged to have an energy setting dependent on the desired depth of effect. Alternatively, the thermal energy source is operated over at least two passes over the skin surface, the energy levels of the passes being chosen dependent on the desired depth of effect.

In either case, the energy setting of the thermal energy source may be such as to create vacuolation on the first pass. In the latter case, the energy setting of the thermal energy source may be such as not to create vacuolation on the first pass, thereby enabling a second pass without removing the treated skin.

Preferably, the energy setting of the thermal energy source is such as to preserve the integrity of the epidermis as a biological dressing.

Preferably, the thermal energy source is operated so that a line of cleavage occurs within the skin 2 to 5 days following treatment, the line of cleavage occurring between said first and second regions. In one particular case, the operation of the thermal energy source may be such as to form a line of cleavage from 2 to 3 cells deep in the stratum corneum of the superficial epidermis and the upper dermis.

Advantageously, the operation of the thermal energy source is such that the tissue in the first region is sloughed tissue. In this case, the sloughed tissue is removed once a new epidermis has been substantially generated in the region of the line of cleavage.

Preferably, the tissue below the line of cleavage in said second region includes the lower epidermis, the basal membrane and the DE Junction. More preferably, at least the thermally-modified basal membrane and the DE Junction are regenerated.

In one particular case, the line of cleavage forms below areas of solar elastosis, such that the solar elastosis and deranged fibroblasts are sloughed.

Preferably, the operation of the thermal energy source is such as to denature dermal collagen in the second region.

Preferably, the tissue in said second region undergoes a regenerative process following regeneration of the epidermis.

In this case, the reticular architecture of the dermis is regenerated in whole, or in part, by fibroblasts less exposed to the effects of UV radiation.

The collagen architecture and/or elastin architecture and/or the GAGS of the dermis is regenerated in whole, or in part, by fibroblasts less exposed to the effects of UV radiation.

Preferably, the healing process is such that risk of scarring and hypo pigmentation is substantially eliminated.

Advantageously, a progressive improvement in skin changes associated with ageing and photodamage occur over a period of between 6 and 12 months following treatment.

Also disclosed as a source of thermal energy is an instrument having an electrode connected to a power output device, and wherein the power output device is operated to create an electric field in the region of the electrode; a flow of gas is directed through the electric field to generate, by virtue of the interaction of the electric field with the gas, a plasma; the plasma is directed onto the tissue for a predetermined period of time; and the power transferred into the plasma from the electric field is controlled so as to desiccate at least a portion of the dermis with vapour pockets formed in dermis cells.

Preferably, the power output device is operated to deliver discrete pulses of heat of millisecond duration.

Advantageously, the pulses have a duration in the range of from about 0.5 milliseconds or 2 milliseconds to about 100 milliseconds, and preferably a duration in the range of from about 4.5 to about 15.4 milliseconds.

Conveniently, the power output device is operated to deliver energy in the range of from about 1 Joule to about 4 Joules for an instrument having a first predetermined nozzle diameter, and to deliver energy in the range of from less than 0.5 Joules to about 2.0 Joules for an instrument having a second predetermined diameter that is less than the first predetermined diameter.

Preferably, the first predetermined diameter is substantially 5mm and the second predetermined diameter is substantially 1.5mm.

The thermal energy may be delivered to the tissue from a thermal energy source as a jet of fluid having stored heat energy at the tissue surface, the jet of fluid typically comprising a jet of ionised diatomic gas.

The invention will now be described by way of example with reference to the drawings in which:
Figure 1 is a diagrammatic view of a tissue treatment system in accordance with the invention;
Figure 2 is a longitudinal cross-section of a tissue treatment instrument forming part of the system of Figure 1, housed in an instrument holder;
Figure 3 is a block diagram of a radio frequency generator for use in the system of Figure 1;
Figure 4 is a cut-away view of the tissue treatment instrument and a calibration device of the system of Figure 1, the instrument located in the instrument holder;
Figure 5 is a diagrammatic representation of the tissue treatment instrument and the calibration device;
Figure 6 is an exploded view of the calibration device;
Figure 7 is a diagram showing an oscilloscope trace of a transducer output signal; and
Figure 8 is a flow diagram illustrating the principle of an energy level compensation method.

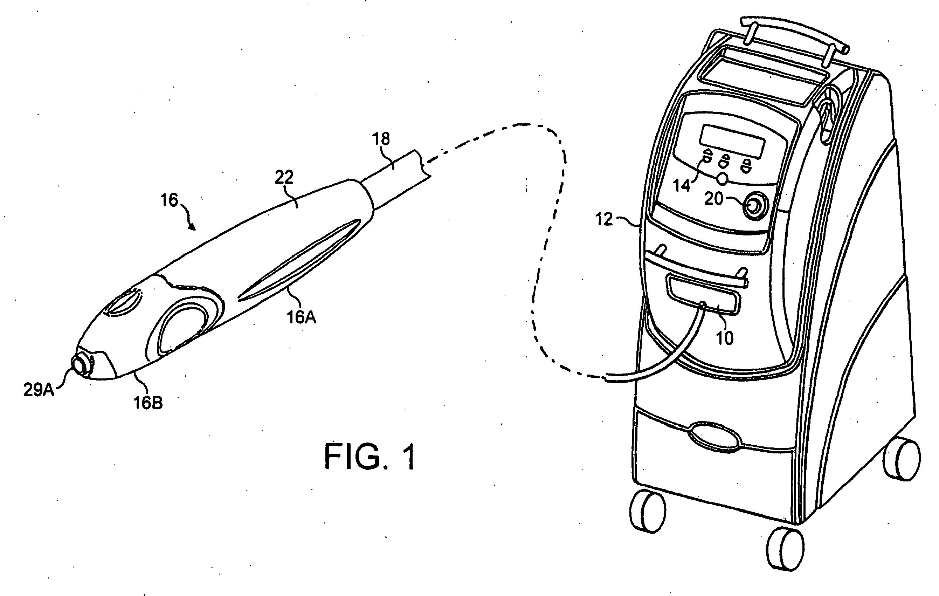

Referring to Figure 1, a tissue treatment system in accordance with the invention has a generator 10 mounted in a floor-standing generator housing 12 and having a user interface 14 for setting the generator to different energy level settings. A handheld tissue treatment instrument 16 is connected to the generator by means of a cord 18. The instrument 16 comprises a re-usable handpiece part 16A and a disposable nose assembly 16B.

The generator housing 12 has an instrument holder 20 for storing the instrument when not in use.

The cord 18 includes a coaxial cable for conveying r.f. energy from the generator 10 to the instrument 16, and a gas supply pipe for supplying nitrogen gas from a gas reservoir or source (not shown) inside the generator housing 12. At its distal end, the cord 18 passes into the casing 22 of the re-usable handpiece part 16A

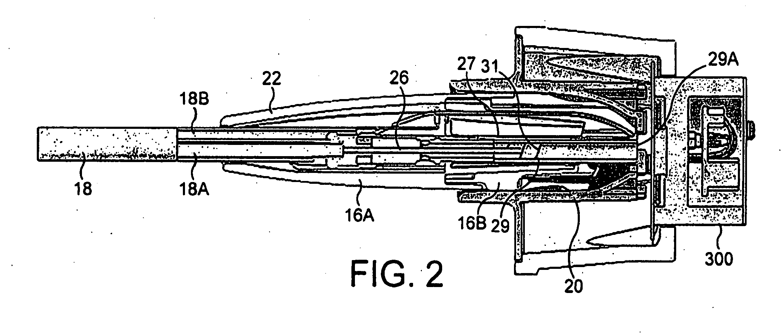

In the re-usable handpiece 16A, the coaxial cable 18A is connected to inner and outer electrodes 26 and 27, as shown in Figure 2, thereby coupling the electrodes to the generator to receive r.f. power. The inner electrode 26 extends longitudinally within the outer electrode 27. Between them is a gas conduit in the form of a heat-resistant tube 29 (preferably made of quartz) housed in the disposable instrument nose assembly 16B. When the nose assembly 16B is secured to the reusable handpiece part 16A, the interior of the tube 29 is in communication with the gas supply pipe interior, the nose assembly being received within the reusable part such that the inner and outer electrodes 26, 27 are associated with the tube, the inner electrode 26 extending axially into the tube 21 and the outer electrode 27 extending around the outside of the tube 29.

A resonator in the form of a helically wound tungsten coil 31 is located within the quartz tube 29, the coil being positioned such that, when the disposable nose 16B is secured in position in the reusable portion of the handpiece, the proximal end of the coil is adjacent the distal end of the inner electrode 26. The coil is wound such that it is adjacent and in intimate contact with the inner surface of the quartz tube 29.

In use, nitrogen gas is fed by a supply pipe to the interior of the tube 29 where it reaches a location adjacent the distal end of the inner electrode 26. When an r.f. voltage is supplied via the coaxial cable to the electrodes 26 and 27 an intense r.f. electric field is created inside the tube 29 in the region of the distal end of the inner electrode. The field strength is aided by the helical coil which is resonant at the operating frequency of the generator and, in this way, conversion of the nitrogen gas into a plasma is promoted, the plasma exiting as a jet at a nozzle 29A of the quartz tube 29. The plasma jet is directed onto tissue to be treated, the nozzle 29A typically being held a few millimetres from the surface of the tissue.

Following repeated use of the instrument, the quartz tube 29 and its resonant coil 31 require replacement. The disposable nose assembly 16B containing these elements is easily attached and detached from the reusable part 16A of the instrument, the interface between the two components 16A, 16B of the instrument providing accurate location of the quartz tube 29 and the coil 31 with respect to the electrodes 26, 27.

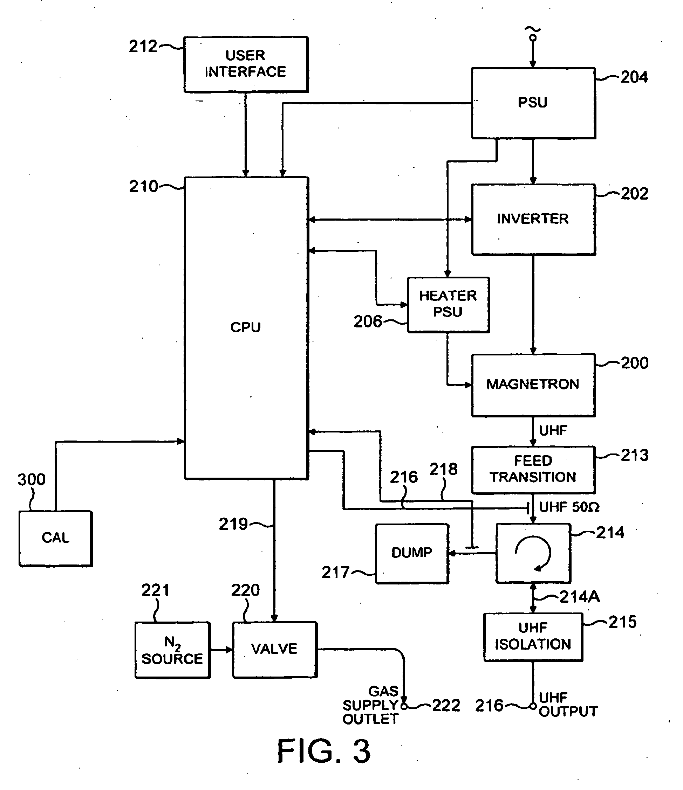

Referring to Figure 3, r.f. energy is generated in a magnetron 200. Power for the magnetron 200 is supplied in two ways, firstly as a high DC voltage for the cathode, generated by an inverter 202 supplied from a power supply unit 204 and, secondly, as a filament supply for the cathode heater from a heater power supply unit 206. Both the high voltage supply represented by the inverter 202 and the filament supply 206 are coupled to a CPU controller 210 for controlling the power output of the magnetron. A user interface 212 is coupled to the controller 210 for the purpose of setting the power output mode, amongst other functions.

The magnetron 200 operates in the high UHF band, typically at 2.475GHz, producing an output on an output line which feeds a feed transition stage 213 for converting the magnetron output to a coaxial 50 ohms feeder, low frequency AC isolation also being provided by this stage. Thereafter, a circulator 214 provides a constant 50 ohms load impedance for the output of the feed transition stage 213. Apart from a first port coupled to the transition stage 213, the circulator 214 has a second port 214A coupled to a UHF isolation stage 215 and hence to the output terminal 216 of the generator for delivering RF power to the handheld instrument 16 (Figure 1). Reflected power is fed from the circulator 214 to a resistive power dump 215. Forward and reflected power sensing connections 216 and 218 provide sensing signals for the controller 210.

The controller 210 also applies via line 219 a control signal for opening and closing a gas supply valve 220 so that nitrogen gas is supplied from the source 221 to a gas supply outlet 222 from where it is fed through the gas supply pipe in the cord 18 to the instrument 16 (Figure 1), when required.

The controller 210 is programmed to pulse the magnetron 200 so that, when the user presses a footswitch (not shown in the drawings), r.f. energy is delivered as a pulsed waveform to the UHF output 216, typically at a pulse repetition rate of between about 1Hz and about 4Hz. A single pulse mode is also provided. The pulses preferably have durations in the range of from 2ms to 100ms. The controller 210 also operates the valve 220 so that nitrogen gas is supplied to the handheld instrument simultaneously with the supply of r.f. energy. Further details of the modes of delivery of r.f. energy are set out in the above-mentioned U.S. Patent No. 6,723,091.

Although r.f. power levels within the generator are monitored by the controller 210, various factors can affect the actual energy or power delivered to the tissue being treated by the plasma jet emerging from the instrument 16 (Figure 1). Accordingly, the delivered energy or power may not correspond accurately to that set by the controller 210, whether according to internal settings programmed into the controller 210 or according to settings performed by the user using the user interface 212.

External factors affecting energy delivery include imperfections in the connecting cable due to, for instance, ageing or physical damage, variations in the gas supply, degradation of the inner electrode 26 (Figure 2) of the handpiece, and degradation or damage to the quartz tube 29.

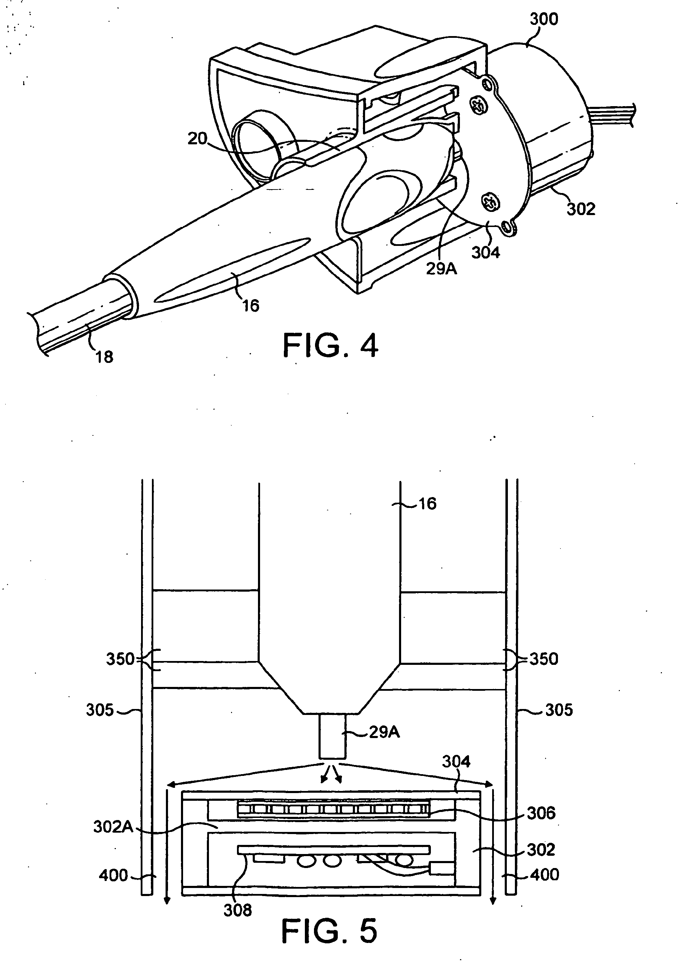

To take account of these factors, the generator includes a calibration device 300, as shown in block form in the generator block diagram of Figure 3 and in the views of Figures 4, 5 and 6.

The calibration device 300 is constituted by a cylindrical calorimeter having a cylindrical body 302 and a planar target element in the form of a thin aluminium plate 304 attached to an end face of the body 302. This calorimeter device 300 is mounted in the generator housing beneath the instrument holder 20, as shown in Figure 4. The internal shape of the instrument holder 20 corresponds to the external shape of a distal portion of the handheld instrument or handpiece 16. In particular, the internal surfaces of the instrument holder 20 are tapered or stepped to locate the nose of the handpiece 16 longitudinally with respect to the target plate 304 of the calibration device 300. The instrument holder 20 is also shaped to locate the handpiece 16 laterally and rotationally, the handpiece distal portion having an asymmetric cross-section which co-operates with corresponding features of the interior of the instrument holder 20 when the handpiece is inserted into the holder. In this way, the position and orientation of the nozzle 29A and, especially, its spacing from the target plate 304 are accurately defined.

In Figure 5, instrument locating features are illustrated diagrammatically as is the calibration device 300. When the instrument 16 is operated in a calibration routine, and when housed in the instrument holder 20, a plasma jet is directed from the nozzle 29A so as to be incident upon a predetermined target surface portion of the target plate 304. Exhausted gas flows radially outwardly from the target surface portion of the target plate which is in registry with the nozzle 29A to escape through circumferential vents 400 between the edge of the target plate 304 and components 305 of the generator housing.

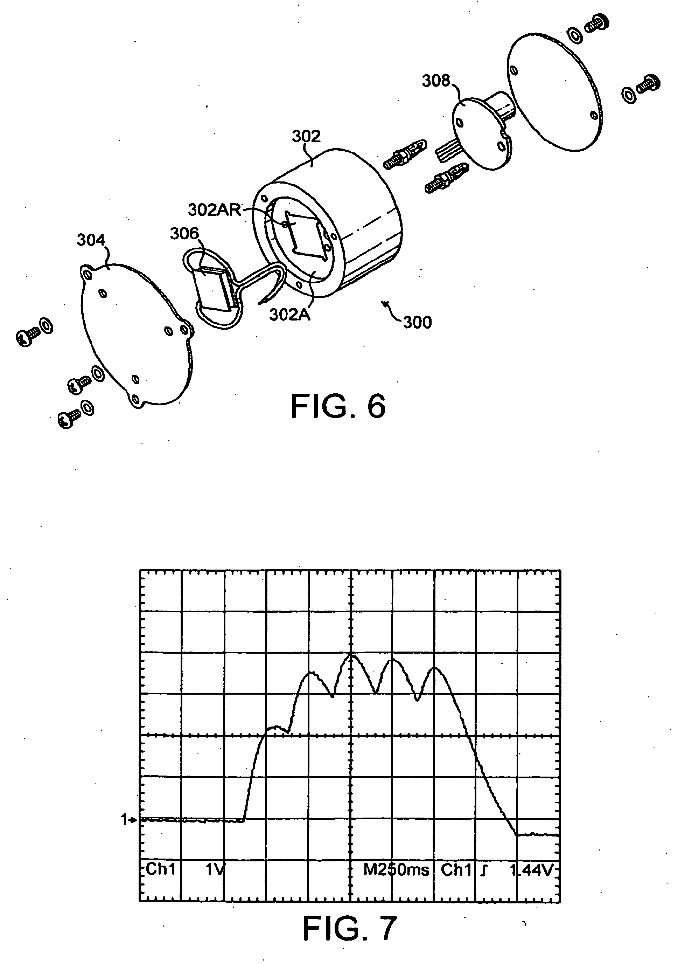

As shown diagrammatically in Figure 5 and in the exploded representation of Figure 6, the calorimeter has an aluminium cylindrical body 302 with an internal heat sink wall 302A upon which is mounted a temperature transducer in the form of a Peltier device 306. A recess 302AR (Figure 6) is formed in the calorimeter heat sink wall 302A for this purpose. The transducer 306 is sandwiched between the heat sink wall 302A and the target plate 304 and is in intimate contact with both. A thermally conductive compound is applied to improve thermal conductivity between the Peltier device 306 and the two surfaces with which it is in contact.

The instrument holder 20 positions the axis of the heat resistant tube 29 centrally with respect to the circular target plate 304 and perpendicularly with respect to the surface of the latter.

The Peltier device 306 has the property (the Seebeck effect) that when there is a difference in temperature between its two faces, a voltage is generated across the device. The voltage is linearly related to the temperature difference. The calibration device 300 includes within its body an electronic circuit 308 which conditions and amplifies the signal from the Peltier device to produce a calibration signal. The output from the circuit is connected to the controller 210 of the generator (see Figure 3) so that the calibration signal can be evaluated. Preferably, the target plate 304 is grounded to the ground of the generator housing.

Operation of the calibration device will now be described with reference to Figure 7.

When the plasma jet impinges on the target plate 304, the target plate is heated. Heat is conducted to the abutting surface of the Peltier device 306. Since the opposite surface of the Peltier device 306 is in thermal contact with the calorimeter body 302 which is at ambient or near-ambient temperature, a temperature differential is created between the two surfaces of the Peltier device 306. This results in a voltage on the output of the Peltier device. Since the temperature rise of the aluminium plate is substantially proportional to the energy delivered, the voltage output of the Peltier device is substantially proportional to delivered energy.

In principle, a single plasma pulse (produced by activating the nitrogen gas supply instrument 16 and simultaneously feeding a single pulse of r.f. energy to the instrument) can be used to characterise the energy delivered by the instrument. In practice, for greater accuracy, it is advantageous to record the effect of a series of plasma pulses and to produce a calibration signal which includes a corresponding plurality of samples of the Peltier device output signal. In the controller 210, a calibration value is computed which is a function of these calibration signal samples, for instance, by averaging to reduce the effects of noise. However, under these circumstances, with a succession of plasma pulses emitted over a period of time (e.g. between half a second and two seconds), the effectiveness of the wall 302A of the calorimeter body as a heat sink diminishes owing to the imperfect dissipation of heat by the calorimeter body 302. As an illustration, if the effect of thermally insulating the Peltier device from the heat sink wall 302A of the calorimeter body is considered, it will be appreciated that this reference surface of the Peltier device, i.e. the surface opposite to that contacting the target plate 304, would be rapidly heated and would eventually become sufficiently hot that the temperature difference between the two surfaces is virtually zero. This would have the effect that the voltage rise with each plasma pulse would be progressively reduced. Even with the Peltier device 306 in intimate thermal contact with the calorimeter body wall 302A, this effect is observed to some extent and, indeed, is visible in the oscilloscope trace of Figure 7.

Referring to Figure 7, an exemplary voltage signal from the Peltier device is shown, being the output observed when five plasma pulses are fired at the target plate 304 from the instrument 16 in the instrument holder 20 (Figure 4). The repetition rate of the pulses in this instance is about 4Hz. The Peltier device output signal resulting from the first pulse of the pulse train appears on the left hand side of the oscilloscope display in Figure 7. Although each plasma pulse delivers the same nominal amount of energy, it is seen that the corresponding signal peak for each pulse is different as a consequence of the progressive heating of the calorimeter body wall 302A (Figure 6).

In this embodiment, the conditioning and amplifying circuit 308 is adjusted so that a plasma pulse equivalent to an energy pulse of 4 joules results in an output voltage change in the region of 1 to 10 volts. Voltages within this range are chosen to avoid electrical interference problems as far as possible. The scaling of voltage with respect to energy is otherwise largely arbitrary and is selected to suit the requirement of interfacing with the CPU of the controller 110 of the generator.

Since the difference in the voltage level for each of the five plasma pulses, as seen in Figure 7, is a function of the thermal characteristics of the calorimeter, and for a given calorimeter design these thermal characteristics are fixed, once the response of the calorimeter to a plasma pulse of known energy is defined, the calorimeter can be used to measure energy delivered by the treatment instrument.

In one preferred embodiment, the controller 210 (Figure 3) samples the peaks of the second, third and fourth Peltier device output pulses and processes these by averaging the peak levels, and compares the resulting value with a stored expected value. In an alternative preferred embodiment the peak level associated with the third output pulse is sampled.

Consistency of results is achieved because the instrument holder 20 shown in Figure 4 avoids misalignment of the instrument 16 with respect to the target plate 304. Any misalignment may result in variation in the "stand-off" distance from the plasma exit nozzle 29A and the target plate 304, the radial offset of the plasma axis with respect to the centre of the Peltier device, and the axis not being perpendicular to the target plate.

Providing the instrument holder 20 is suitably shaped, the calibration device can be operated with the required performance and immunity to misalignment when used with different instruments having different exit nozzle diameters.

One feature that reduces the effect of any misalignment which may be the consequence of manufacturing tolerances or variations in the manner in which the instrument is inserted and positioned in the instrument holder, the Peltier device has a sensing area greater than the nominal width of the plasma jet from the instruments to be calibrated. In addition, the Peltier device has a distributed array of individual temperature sensing elements to reduce sensitivity to radial misalignment.

The relatively large area of the Peltier device 306 also increases the likelihood of the whole of the plasma jet impinging upon the target plate within the sensing area of the Peltier device. Therefore, given that the plasma jet is commonly not well collimated and diverges on exit from the nozzle 29A, the calibration device is relatively insensitive to variations in stand-off distance of the nozzle 29A from the target plate 304.

Likewise, the relatively large area of the Peltier device 306 contributes to immunity to variations in the alignment of the plasma jet axis with respect to the target plate 304.

The preferred embodiment of the system in accordance with the invention is configured to define a semi-automatic calibration procedure.

Prior to treatment, the user is required to fit the treatment instrument 16 into the instrument holder 20 (Figure 4). In response to electrical sensing of correct fitment of the instrument in the holder 20 or in response to indication from the user, e.g. in the form of a key-press confirmation, a signal is provided to the user requesting operation of the footswitch to activate the instrument. Where the instrument has a disposable portion, the system may also require confirmation that this portion has been fitted.

Upon activation of the footswitch, the generator and automatic control of the CPU in the controller 210 (Figure 3) causes the generation of the predetermined number (five) of plasma pulses at a specified interval between each consecutive pulse. In the preferred embodiment, the pulse repetition interval is 250ms. The calibration signal from the calibration device is then processed by the controller 210 and the resulting calibration value compared with an expected value for the particular instrument in question (or particular nozzle where more than one type of nozzle may be fitted).

Depending on the size and nature of any deviation from the expected value, the generator may:
a) Make a small adjustment - 'automatic calibration' or self calibration - to the r.f. power level to compensate for a small deviation (e.g., up to or less than 10% or otherwise compatible with the system as a whole meeting the safety and efficacy requirements of a medical device) from a nominal energy setting. This enables variations in the performance of the system and disposable nozzle to be compensated for automatically and gives greater accuracy in energy delivery than would otherwise be the case. Note that excessive output power adjustment can cause overdose or critical limiting circuitry that operates independently of the software control system to detect a fault condition and so prevent further operation.
b) Determine that ageing or other effects have degraded the generator and/or its accessories such that maintenance or another servicing function is likely to be required but that, otherwise, the output level can be sufficiently well compensated for such that the energy delivery accuracy is as required and consistent with the safety and efficacy of the system. This may be communicated to the user through a message or other indicator.
c) Determine that the nozzle or other system part that may be readily changed by the user is faulty and, on confirmation of replacement, cause the test to be repeated.
d) Determine that there is a fault caused, for example, by damage to the r.f. cable between the generator and handpiece, that will cause incorrect operation and incorrect delivery of energy to the patient. In this case the generator will halt further operation (unless reset, in which case the test must be performed before treatment). An appropriate message is then displayed to indicate the fault condition to the user.

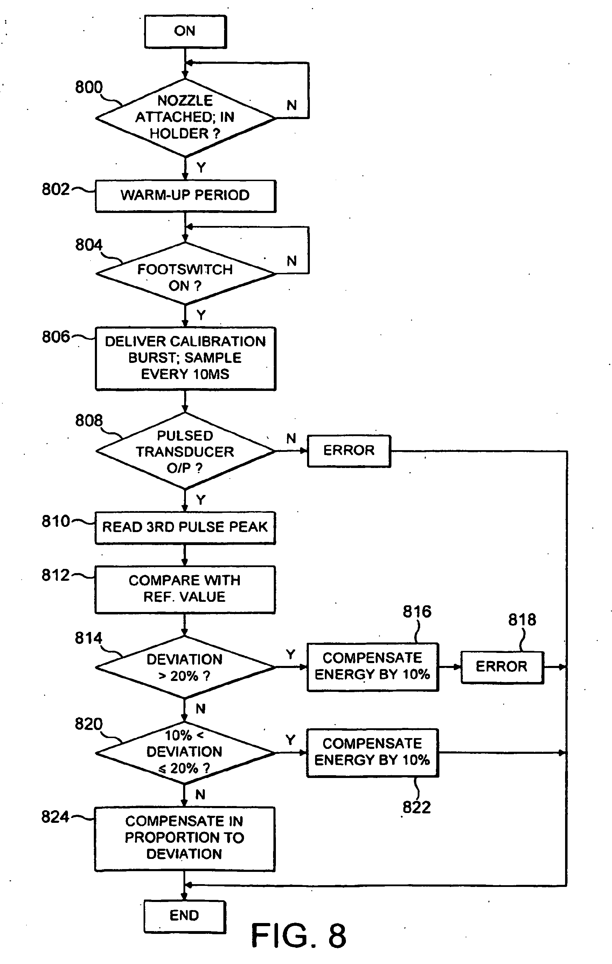

With regard to the calibration and adjustment of the system, the principle steps performed by the controller 210 in combination with the calibration device 300 include adjusting the generator in response to a calibration measurement to compensate for a deviation in an expected energy measurement value, thereby establishing a new energy delivery setting for a treatment session which begins with the calibration procedure. In the preferred system, the energy level is adjusted substantially in proportion to the deviation of the measurement from the expected value. This may be performed up to the limit or limits of a predetermined adjustment range, e.g. 10% above and/or 10% below the expected value. In particular, the preferred system performs a sequence of steps as shown in the flow diagram of Figure 8.

Referring to Figure 8, an initial self-check step 800 involves the user confirming that the handpiece has a nozzle attached and that the handpeice and the nozzle have been placed in the instrument holder 20 (Figure 1). After a warm-up period 802, the controller detects operation of the footswitch for at least a minimum predetermined time (step 804). A calibration energy burst is then delivered, the plasma jet impinging upon the target element, and the Peltier device output signal is sampled every 10ms (step 806). Providing a pulsed output signal is detected (step 808), the peak level of a predetermined output signal pulse, in this case the third pulse, is measured (step 810) and compared with an expected or reference value (step 812).

If the comparison indicates a deviation of the output signal level greater than 20% from the expected value (step 814) the generator output is adjusted by 10% as a part-compensation (step 816), and an error indication provided (step 818).

If the deviation from the expected value is less than or equal to 20% (step 820), but greater than 10%, the generator output is adjusted by 10% (step 822) but no error signal is provided.

If the deviation is less than 10%, the energy level setting of the generator is adjusted in proportion to the deviation and in a direction so as to compensate for the deviation (step 824) (i.e. by increasing the generator output if the measured value is lower than the expected value or by decreasing the generator output if the measured value is higher than the expected value.

It will be appreciated, therefore, that in the preferred embodiment, energy level compensation is performed on a linear basis up to a predetermined maximum adjustment, in this case 10% in energy terms. Greater deviations of the measured value from the expected value give rise to a maximum energy level adjustment equal to the predetermined maximum adjustment, i.e. 10%, thereby part-compensating for the deviation.

As previously stated, it is a system requirement that the energy delivery be checked before patient treatment. The preferred embodiment allows the user to perform a calibration check at a later time.

In addition, on change of nozzle (either initiated by the user or through other means) the user is required by the generator to check the performance of the instrument.

In the preferred embodiment, a device having an array of semiconductor elements (the Peltier device) is used as a temperature sensing transducer. It is, as an alternative, also possible to use a thermocouple or an array of thermocouples, or one or more thermistors.

The described and illustrated calibration device forms part of the generator and is mounted to the generator housing. However, the device could be used externally and may be connected to the generator.

Other systems may include systems in which heating energy is delivered to the tissue from a source having a low thermal time constant. Typically, treatment energy can be delivered in pulses of very short duration (typically 0.5 to 100ms) and without reliance on an intermediary conversion from one kind of energy to another such as a chromophore in laser energy and tissue resistivity in radio frequency energy.

In use, the instrument 16 is passed over the surface of tissue to be cosmetically treated, with the nozzle 29a typically being held a few millimetres from the surface of the tissue. The pulse duration and energy levels are chosen so as to form first and second adjacent regions of thermally-modified tissue in the region of the DE Junction. The first, upper region is termed a zone of thermal damage, having a thermal modification which is greater than that of the second, lower region. The thermally damaged zone is thermally modified to an extent that it separates from the second region some days after the delivery of the thermal energy. Following separation of the first damaged region, the epidermis and the upper region of the dermis regenerate naturally.

A benefit of using a diatomic plasma is that it is able to deliver a relatively large amount of energy which causes heating in a short period of time. This enables delivery in discreet pulses of millisecond duration, and is in contrast to heat conduction from a merely hot gas. In the preferred embodiment, energy from 1 Joule to 4 Joules is delivered in a period of 4.5 to 15.4 milliseconds respectively for a nozzle with an exit diameter of 5 millimetres, and delivers from less than 0.5 Joules up to 2 Joules in the same period for a nozzle with an exit diameter of less than 1.5 millimetres. Experiments have shown that useful clinical effects are achieved with yet longer pulses extending to 50 milliseconds, and further analysis shows extension up to 100 milliseconds or more will provide useful effects. In addition, the pulse width may be shortened to deliver the same, or otherwise similar, useful heating energy. Plasma pulses as short as 0.5 milliseconds have been produced with the system described above.

Another benefit is that oxygen is purged from the skin surface by the plasma and flow of inert gas that follows immediately following a plasma pulse. As a result, the oxidative carbonisation that often occurs at the skin surface on application of thermal energy is avoided, leaving a desiccated intact epithelium with minor structural alteration.

This minor structural alteration is nonetheless important in providing yet another benefit of the invention, as it changes the thermal characteristics of the epidermis at higher energy settings. Following a single pass of plasma over the skin surface at an energy setting greater than 2 Joules, the epidermal cells at the basal membrane are heated to a degree that produces vacuolation of the cellular contents. This produces a natural insulator limiting the absorption and depth of penetration of energy from subsequent passes. This is a beneficial safety feature that avoids the risk of excessive damage by inadvertent application of multiple passes to the same site on the skin surface.

Alternatively, when using energy pulses at or below 2 Joules, then the vacuolation is not observed, and the treated skin is still capable of absorbing the thermal energy of a second pass, by changing the energy in the second pass using either a narrow nozzle to focus the plasma or a higher energy setting will have an additive effect. The benefit of using a narrow nozzle embodiment is that the focused energy can be directed onto specific areas of the skin surface such as deeper wrinkles.

For example, if the skin is subjected to two passes of 4 Joules, then the depth of thermal effect is only 10-20% greater than with a single pass of 4 Joules. Alternatively, if the skin is first treated with 2 Joules, then with a second pass of 4 Joules then the effect will be consistent with a single pass with 6 Joules. Part of this benefit also relates to the water content of the skin, particularly the upper layers of the epidermis following pre-treatment with a topical anaesthetic.

Through experimentation with the invention, it has become clear that the depth of effect changes by up to 50% depending on the hydration of the upper layers of the epidermis following application of a topical anaesthetic. Topical anaesthetics include a hydrating component, as they rely on hydration of the superficial epidermis for the penetration of the anaesthetic agent through the skin. This changes the absorption of pure thermal energy, whereby a larger proportion of the energy is dissipated in the superficial epidermis, reducing the depth of penetration into the dermis. If no anaesthesia or tumescent subcuticular anaesthesia is employed, then the depth of dermal penetration for a given energy setting can be doubled. A pre-treatment with 2 Joules produces sufficient desiccation of the superficial epidermis, following use of topical anaesthesia, that an equivalent depth of effect can be produced with the second pass to that achieved with no anaesthesia or tumescent subcuticular anaesthesia.

The reason for using a diatomic plasma which delivers a relatively large amount of energy in a short period of time is that the irreversible clinical effects (the thermal modification and thermal damage of the tissue) occur over tissue depths that result in the desired clinical effects, whilst avoiding any undesired clinical effects. If the heating energy is delivered over too long a time, the effects of convection from the skins surface and conduction into the underlying tissue will be such that no significant temperature rise results. On the other hand, if the time is too short, then irreversible effects (such as water vaporising) at or near the skins surface will carry away otherwise useful heating energy.

## Claims

1. A tissue treatment system including a radio frequency (r.f.) generator (10), a treatment instrument (16) connectible to the generator and to a source (221) of ionisable gas and operable to produce a plasma jet at a nozzle (29A) of the instrument when supplied with the ionisable gas and energised by the generator, and a calibration device which comprises:
a target element (304) having a target surface area,
a transducer (306) arranged to produce an electrical signal indicative of a temperature change of the target element within the target surface area,
a receptacle (20) for locating the nozzle of the instrument in juxtaposition with the target surface area whereby, in operation of the instrument when in the receptacle, the plasma jet is incident upon the target surface area to cause the transducer to generate a calibration signal for adjusting the energy output of the generator, and an output connected to the generator for coupling the calibration signal to the generator,
the generator including an energy output adjuster (210) for adjusting the r.f.
energy output of the generator, the adjuster being configured to adjust the r.f. energy output in response to the calibration signal.

2. A system according to claim 1, wherein the adjuster (210) forms part of a controller which is configured to perform an automatic calibration process in which the generator (10) is operated to feed a predetermined r.f. energy waveform to the instrument (16) to cause generation of a calibration plasma jet directed at the target surface area of the calibration device (300), the calibration signal generated as a result of the calibration jet heating the target element (304) is processed to produce a calibration value which is then compared with a reference value, and the adjuster is operated automatically in response to a deviation of the calibration value from the reference value to set the r.f. energy output for subsequent operation of the system.

3. A system according to claim 2, wherein the predetermined r.f. energy waveform comprises a plurality of pulses of r.f energy.

4. A system according to claim 3, wherein the calibration value is a function combining the levels of successive pulses which are generated by the transducer (306) and correspond to the pulses of r.f. energy.

5. A system according to any preceding claim, wherein the target element (304) is a metallic plate and the transducer (306) is in contact with the plate.

6. A system according to any preceding claim, wherein the transducer (306) is arranged to produce an electrical signal representative of a difference in temperature between first and second elements of the transducer.

7. A system according to any of claims 1 to 5, wherein the transducer has a measurement surface and a reference surface, which surfaces are spaced apart, and wherein the transducer is operable to produce an electrical signal which is substantially linearly related to the difference between a temperature sensed at the measurement surface and a temperature sensed simultaneously at the reference surface.

8. A system according to any of claims 1 to 5, wherein the target element (304) comprises a first transducer element, wherein the calibration device has a second transducer element spaced from the first transducer element, and wherein the system is arranged to perform a calibration process during which the temperature existing at the first transducer element is compared with the temperature existing at the second transducer element to produce the said electrical signal.

9. A system according to any of claims 1 to 4, wherein the calibration device (300) comprises a calorimeter having a metallic plate (304) forming the target element, a heat sink portion (302) underlying the metallic plate, and a temperature difference sensor (306), located between the metallic plate and the heat sink portion and arranged to produce an electrical output signal indicative of the difference in temperature between the metallic plate and the heat sink portion.

10. A system according to claim 9, wherein the sensor (306) comprises a Peltier device.

11. A system according to claim 5, claim 9 or claim 1, wherein the transducer (306) has an area greater than the cross-sectional area of the plasma jet when the instrument (16) is operated in its calibration location.

12. A system according to claim 11, wherein the transducer (306) comprises a plurality of temperature sensitive elements distributed in an array.

13. A system according to claim 1, wherein the generator (10) is operable in a calibration mode to cause a burst of r.f. energy to be delivered to the instrument (16) with a predetermined waveform and duration so as to cause the instrument, when supplied with the said gas, to generate a calibration plasma jet, and wherein the calibration device is arranged automatically to evaluate the said electrical signal produced when the calibration plasma jet is incident on the target surface area to generate an adjustment signal for adjusting the energy output of the generator.

14. A system according to claim 13, wherein the calibration device is arranged to evaluate the level of the said electrical signal at a predetermined time after commencement of the burst of r.f. energy.

15. A system according to claim 13, wherein the generator (10) is operable to produce the said burst as a plurality of pulses and the calibration device is arranged to evaluate the level of the said electrical signal after the application of a predetermined number of pulses.

16. A system according to claim 13, wherein the transducer (306) is a temperature difference sensor and the calibration device is arranged to evaluate the level of the said electrical signal when it reaches a maximum level.

17. A system according to claim 13, wherein the generator (10) is operable to produce the said burst as a succession of pulses, the transducer (306) is a temperature difference sensor, and the calibration device is arranged to evaluate the level of the said electrical signal when it reaches a peak level associated with the *n*^{th} pulse occurring in the said succession of pulses, n being a predetermined integer.

18. A system according to any preceding claim, wherein:
the generator (10) is contained within a generator housing (12) to which the treatment instrument (16) is coupled by a cable (18) for conveying r.f. energy from the generator to the instrument;
the calibration device is in the generator housing and has an associated instrument holder (20) which opens out on a front section of the housing for receiving the instrument and which is shaped to locate the instrument longitudinally and transversely with respect to the target element (304).

19. A method of calibrating a tissue treatment system which includes a radio frequency (r.f.) generator (10), a treatment instrument (16) connected to the generator and to a source (221) of ionisable gas, and a calibration device comprising a target element (304), a temperature sensing transducer (306), a receptacle (20) for locating a nozzle (29A) of the instrument in juxtaposition with a target surface area of the target element at a pre-defined location with respect thereto, and an output coupled to the generator, wherein the method comprises:
placing the treatment instrument in the receptacle;
causing gas to be supplied to the instrument and simultaneously causing a burst of r.f. energy to be delivered to the instrument with a predetermined waveform and duration so as to generate a plasma jet exiting the nozzle and incident upon the target element;
automatically evaluating a calibration signal generated by the calibration device at the said output and representative of an output of the transducer resulting from heating of the target element by the plasma jet; and
setting the r.f. energy output level of the generator according to the evaluation of the calibration signal.

## Patentansprüche

1. Gewebebehandlungssystem, das einen Hochfrequenz(RF)-Generator (10) umfasst, ein Behandlungsinstrument (16), welches mit dem Generator und mit einer Quelle (221) für ionisierbares Gas verbunden werden kann und derart betrieben werden kann, dass ein Plasmastrahl an einer Düse (29A) des Instruments erzeugt wird, wenn diesem ionisierbares Gas zugeführt wird und es durch den Generator mit Strom versorgt wird, sowie eine Kalibrierungsvorrichtung, welche umfasst:
ein Zielelement (304) mit einem Zieloberflächenbereich,
einen Messwandler (306), der dafür ausgelegt ist, ein elektrisches Signal zu erzeugen, das für eine Temperaturänderung des Zielelements in dem Zieloberflächenbereich kennzeichnend ist,
einen Stecksockel (20) zum Positionieren der Düse des Instruments in Gegenüberstellung zu dem Zieloberflächenbereich, wodurch beim Betrieb des Instruments, wenn sich dieses in dem Stecksockel befindet, der Plasmastrahl auf den Zieloberflächenbereich auftrifft und bewirkt, dass der Messwandler ein Kalibrierungssignal zum Anpassen der Ausgangsenergie des Generators erzeugt, und wobei ein Ausgang mit dem Generator verbunden ist, um das Kalibrierungssignal in den Generator einzukoppeln,
wobei der Generator eine Ausgangsenergie-Einstelleinrichtung (210) zum Anpassen der RF-Ausgangsenergie des Generators umfasst, wobei die Einstelleinrichtung dafür konfiguriert ist, die RF-Ausgangsenergie in Reaktion auf das Kalibrierungssignal anzupassen.

2. System nach Anspruch 1, wobei die Einstelleinrichtung (210) Teil einer Steuerung ist, welche dafür konfiguriert ist, einen automatischen Kalibrierungsvorgang auszuführen, bei welchem der Generator (10) derart betrieben wird, dass er dem Instrument (16) eine vorgegebene RF-Energiewellenform zuführt, um die Erzeugung eines Kalibrierungsplasmastrahls zu bewirken, der auf den Zieloberflächenbereich der Kalibrierungsvorrichtung (300) gerichtet wird, wobei das im Ergebnis der Aufheizung des Zielelements (304) durch den Kalibrierungsstrahl erzeugte Kalibrierungssignal verarbeitet wird, um einen Kalibrierungswert zu erzeugen, welcher dann mit einem Referenzwert verglichen wird, und wobei die Einstelleinrichtung in Reaktion auf eine Abweichung des Kalibrierungswertes von dem Referenzwert automatisch derart betrieben wird, dass die RF-Ausgangsenergie für nachfolgende Betriebsvorgänge des Systems eingestellt wird.

3. System nach Anspruch 2, wobei die vorgegebene RF-Energiewellenform mehrere RF-Energieimpulse umfasst.

4. System nach Anspruch 3, wobei der Kalibrierungswert eine Funktion der Kombination von Pegeln aufeinander folgender Impulse, die von dem Messwandler (306) erzeugt werden und den RF-Energieimpulsen entsprechen, ist.

5. System nach einem der vorhergehenden Ansprüche, wobei das Zielelement (304) eine Metallplatte darstellt und der Messwandler (306) in Kontakt mit der Platte steht.

6. System nach einem der vorhergehenden Ansprüche, wobei der Messwandler (306) dafür ausgelegt ist, ein elektrisches Signal zu erzeugen, das für eine Temperaturdifferenz zwischen einem ersten und einem zweiten Element des Messwandlers kennzeichnend ist.

7. System nach einem der Ansprüche 1 bis 5, wobei der Messwandler eine Messoberfläche und eine Bezugsoberfläche aufweist, wobei diese Oberflächen im Abstand zueinander angeordnet sind, und wobei der Messwandler dazu dient, ein elektrisches Signal zu erzeugen, das im Wesentlichen linear proportional zu der Differenz zwischen einer auf der Messoberfläche gemessenen Temperatur und einer gleichzeitig auf der Bezugsoberfläche gemessenen Temperatur ist.

8. System nach einem der Ansprüche 1 bis 5, wobei das Zielelement (304) ein erstes Wandlerelement umfasst, wobei die Kalibrierungsvorrichtung ein zweites Wandlerelement im Abstand von dem ersten Wandlerelement aufweist und wobei das System dafür ausgelegt ist, einen Kalibrierungsprozess auszuführen, während dem die an dem ersten Wandlerelement auftretende Temperatur mit der an dem zweiten Wandlerelement auftretenden Temperatur verglichen wird, um das elektrische Signal zu erzeugen.

9. System nach einem der Ansprüche 1 bis 4, wobei die Kalibrierungseinrichtung (300) ein Kalorimeter umfasst, welches eine Metallplatte (304) aufweist, die das Zielelement bildet, einen Wärmesenkeabschnitt (302), der unter der Metallplatte liegt, sowie einen Temperaturdifferenzsensor (306), der zwischen der Metallplatte und dem Wärmesenkeabschnitt angeordnet ist und dafür ausgelegt ist, ein elektrisches Ausgangssignal zu erzeugen, das für die Temperaturdifferenz zwischen der Metallplatte und dem Wärmesenkeabschnitt kennzeichnend ist.

10. System nach Anspruch 9, wobei der Sensor (306) ein Peltier-Element umfasst.

11. System nach Anspruch 5, Anspruch 9 oder Anspruch 1, wobei der Messwandler (306) eine Fläche aufweist, die größer als die Querschnittsfläche des Plasmastrahls ist, wenn das Instrument (16) an seiner Kalibrierungsposition betrieben wird.

12. System nach Anspruch 11, wobei der Messwandler (306) mehrere temperaturempfindliche Elemente umfasst, die in einem Feld verteilt sind.

13. System nach Anspruch 1, wobei der Generator (10) in einem Kalibrierungsmodus dazu dient, einen RF-Energiestoß zu bewirken, der mit einer vorgegebenen Wellenform und Dauer dem Instrument (16) zugeführt wird, sodass bewirkt wird, dass das Instrument, wenn diesem das Gas zugeführt wird, einen Kalibrierungsplasmastrahl erzeugt, und wobei die Kalibrierungsvorrichtung dafür ausgelegt ist, das elektrische Signal, welches erzeugt wird, wenn der Kalibrierungsplasmastrahl auf den Zieloberflächenbereich auftrifft, automatisch zu bewerten, um ein Einstellsignal zum Anpassen der Ausgangsenergie des Generators zu erzeugen.

14. System nach Anspruch 13, wobei die Kalibrierungsvorrichtung dafür ausgelegt ist, den Pegel des elektrischen Signals zu einem vorgegebenen Zeitpunkt nach Beginn des RF-Energiestoßes zu bewerten.

15. System nach Anspruch 13, wobei der Generator (10) dazu dient, den Energiestoß als Mehrzahl von Impulsen zu erzeugen, und wobei die Kalibrierungsvorrichtung dafür ausgelegt ist, den Pegel des elektrischen Signals nach dem Anlegen einer vorgegebenen Anzahl von Impulsen zu bewerten.

16. System nach Anspruch 13, wobei der Messwandler (306) ein Temperaturdifferenzsensor ist und die Kalibrierungsvorrichtung dafür ausgelegt ist, den Pegel des elektrischen Signals zu bewerten, wenn dieses einen maximalen Pegel erreicht.

17. System nach Anspruch 13, wobei der Generator (10) dazu dient, den Energiestoß als Abfolge von Impulsen zu erzeugen, wobei der Messwandler (306) ein Temperaturdifferenzsensor ist und wobei die Kalibrierungsvorrichtung dafür ausgelegt ist, den Pegel des elektrischen Signals zu bewerten, wenn dieses einen Spitzenpegel erreicht, welcher dem n-ten Impuls zugeordnet ist, der in der Impulsabfolge auftritt, wobei n eine vorgegebene ganze Zahl ist.

18. System nach einem der vorhergehenden Ansprüche, wobei:
der Generator (10) in einem Generatorgehäuse (12) angeordnet ist, mit welchem das Behandlungsinstrument (16) über ein Kabel (18) verbunden ist, um RF-Energie von dem Generator zu dem Instrument zu übertragen,
wobei sich die Kalibrierungsvorrichtung in dem Generatorgehäuse befindet und einen zugehörigen Instrumentenhalter (20) aufweist, der an einem vorderen Abschnitt des Gehäuses offen ist, um das Instrument aufzunehmen, und wobei dieser eine solche Gestalt aufweist, dass das Instrument in Längsrichtung und Querrichtung in Bezug auf das Zielelement (304) positioniert wird.

19. Verfahren zum Kalibrieren eines Gewebebehandlungssystems, welches einen Hochfrequenz(RF)-Generator (10), ein mit dem Generator und mit einer Quelle (221) für ionisierbares Gas verbundenes Behandlungsinstrument (16) und eine Kalibrierungsvorrichtung umfasst, welche ein Zielelement (304), einen Temperaturmesswandler (306), einen Stecksockel (20) zum Positionieren einer Düse (29A) des Instruments in Gegenüberstellung zu einem Zieloberflächenbereich des Zielelements an einer vorgegebenen Position in Bezug auf dieses sowie einen mit dem Generator gekoppelten Ausgang umfasst, wobei das Verfahren umfasst:
Anordnen des Behandlungsinstruments in dem Stecksockel;
Bewirken, dass dem Instrument Gas zugeführt wird und gleichzeitig Bewirken, dass dem Instrument ein RF-Energiestoß mit einer vorgegebenen Wellenform und Dauer zugeführt wird, um so einen Plasmastrahl zu erzeugen, der aus der Düse austritt und auf das Zielelement auftrifft;
automatisches Bewerten eines Kalibrierungssignals, das durch die Kalibrierungsvorrichtung an dem Ausgang erzeugt wird und ein Ausgangssignal des Messwandlers repräsentiert, das sich aus der Aufheizung des Zielelements durch den Plasmastrahl ergibt; und
Einstellen des RF-Energieausgangspegels des Generators entsprechend der Bewertung des Kalibrierungssignals.

## Revendications

1. Système de traitement de tissus comprenant un générateur de radiofréquences (r.f.) (10), un instrument de traitement (16) pouvant être connecté au générateur et à une source (221) de gaz ionisable et pouvant être actionné pour produire un jet de plasma au niveau d'une buse (29A) de l'instrument, lorsqu'il est alimenté en gaz ionisable et excité par le générateur, et dispositif de calibrage comprenant :
un élément cible (304) ayant une zone de surface cible,
un transducteur (306) configuré pour produire un signal électrique indicatif d'un changement de température de l'élément cible dans la zone de surface cible,
un logement (20) pour mettre en place la buse de l'instrument en la juxtaposant à la zone de surface cible, moyennant quoi, lors du fonctionnement de l'instrument une lorsqu'il est dans le logement, le jet de plasma heurte la zone de surface cible pour amener le transducteur à générer un signal de calibrage destiné à régler l'énergie en sortie du générateur, et une sortie connectée au générateur pour appliquer le signal de calibrage au générateur,
le générateur intégrant une fonction de réglage d'énergie en sortie (210) destinée à régler l'énergie r.f. en sortie du générateur, cette fonction de réglage étant configurée pour régler l'énergie r.f. en sortie en réponse au signal de calibrage.

2. Système selon la revendication 1, dans lequel la fonction de réglage (210) fait partie d'un contrôleur qui est configuré pour exécuter un processus de calibrage automatique dans lequel le générateur (10) est actionné pour transmettre une forme d'onde d'énergie r.f. prédéterminée à l'instrument (16) afin de déclencher la génération d'un jet de plasma de calibrage dirigé sur la zone de surface cible du dispositif de calibrage (300), le signal de calibrage généré suite au chauffage par le jet de calibrage de l'élément cible (304) est traité pour produire une valeur de calibrage qui est ensuite comparée à une valeur de référence, et la fonction de réglage est actionnée automatiquement en réponse à un écart de la valeur de calibrage par rapport à la valeur de référence afin de régler l'énergie r.f. en sortie pour l'opération suivante du système.

3. Système selon la revendication 2, dans lequel la forme d'onde d'énergie r.f. prédéterminée comprend une pluralité d'impulsions d'énergie r.f.

4. Système selon la revendication 3, dans lequel la valeur de calibrage est une fonction combinant les niveaux des impulsions successives qui sont générées par le transducteur (306) et correspondent aux impulsions d'énergie r.f..

5. Système selon l'une quelconque des revendications précédentes, dans lequel l'élément cible (304) est une plaque métallique et le transducteur (306) est en contact avec cette plaque.

6. Système selon l'une quelconque des revendications précédentes, dans lequel le transducteur (306) est configuré pour produire un signal électrique représentatif d'une différence de température entre les premier et second éléments du transducteur.

7. Système selon l'une quelconque des revendications 1 à 5, dans lequel le transducteur a une surface de mesure et une surface de référence, lesquelles surfaces sont séparées, et dans lequel le transducteur peut être actionné pour produire un signal électrique correspondant de manière sensiblement linéaire à la différence entre une température mesurée à la surface de mesure et une température mesurée simultanément au niveau de la surface de référence.

8. Système selon l'une quelconque des revendications 1 à 5, dans lequel l'élément cible (304) comprend un premier élément de transducteur, dans lequel le dispositif de calibrage a un second élément de transducteur séparé du premier élément de transducteur, et dans lequel le système est configuré pour exécuter un processus de calibrage durant lequel la température mesurée au premier élément de transducteur est comparée à la température mesurée au second élément de transducteur pour produire ledit signal électrique.

9. Système selon l'une quelconque des revendications 1 à 4, dans lequel le dispositif de calibrage (300) comprend un calorimètre ayant une plaque métallique (304) formant l'élément cible, une partie drain thermique (302) sous-jacente à la plaque métallique, et une sonde de différence de température (306), placée entre la plaque métallique et la partie drain thermique et configurée pour produire un signal de sortie électrique indicatif de la différence de température entre la plaque métallique et la partie drain thermique.

10. Système selon la revendication 9, dans lequel le capteur (306) comprend un dispositif de Peltier.

11. Système selon la revendication 5, la revendication 9 ou la revendication 1, dans lequel le transducteur (306) a une surface supérieure à la surface en coupe du jet de plasma quand l'instrument (16) est actionné à son emplacement de calibrage.

12. Système selon la revendication 11, dans lequel le transducteur (306) comprend une pluralité d'éléments sensibles à la température distribués selon un réseau.

13. Système selon la revendication 1, dans lequel le générateur (10) peut fonctionner dans un mode de calibrage afin de déclencher l'émission d'une salve d'énergie r.f. vers l'instrument (16) ayant une forme d'onde et une durée prédéterminées de façon à amener l'instrument, une lorsqu'il est alimenté par ledit gaz, à générer un jet de plasma de calibrage, et dans lequel le dispositif de calibrage est configuré automatiquement afin d'évaluer ledit signal électrique produit lorsque le jet de plasma de calibrage heurte la zone de surface cible pour générer un signal de réglage destiné à régler l'énergie en sortie du générateur.

14. Système selon la revendication 13, dans lequel le dispositif de calibrage est configuré pour évaluer le niveau dudit signal électrique à un moment prédéterminé après le départ de la salve d'énergie r.f.

15. Système selon la revendication 13, dans lequel le générateur (10) peut fonctionner pour produire ladite salve sous forme d'une pluralité d'impulsions et le dispositif de calibrage est configuré pour évaluer le niveau dudit signal électrique après l'application d'un nombre prédéterminé d'impulsions.

16. Système selon la revendication 13, dans lequel le transducteur (306) est une sonde de différence de température et le dispositif de calibrage est configuré pour évaluer le niveau dudit signal électrique quand il atteint un niveau maximal.

17. Système selon la revendication 13, dans lequel le générateur (10) peut être actionné pour produire ladite salve sous la forme d'une séquence d'impulsions, le transducteur (306) est une sonde de différence de température et le dispositif de calibrage est configuré pour évaluer le niveau dudit signal électrique quand il atteint un niveau crête associé à la énième impulsion produite dans ladite séquence d'impulsions, n étant un entier prédéterminé.

18. Système selon l'une quelconque des revendications précédentes, dans lequel :
le générateur (10) est contenu dans un logement de générateur (12) auquel l'instrument de traitement (16) est couplé par un câble (18) destiné à transmettre l'énergie r.f. du générateur à l'instrument ;
le dispositif de calibrage se trouve dans le logement de générateur et a un support d'instrument associé (20) qui s'ouvre sur une section avant du logement destiné à recevoir l'instrument, et qui a une forme lui permettant de mettre en place l'instrument longitudinalement et transversalement par rapport à l'élément cible (304).

19. Procédé de calibrage d'un système de traitement de tissus intégrant un générateur de radiofréquences (r.f.) (10), un instrument de traitement (16) connecté au générateur et à une source (221) de gaz ionisable, et un dispositif de calibrage comprenant un élément cible (304), un transducteur de mesure de température (306), un logement (20) pour mettre en place une buse (29A) de l'instrument en la juxtaposant à une zone de surface cible de l'élément cible, à un emplacement prédéfini par rapport à celle-ci, et une sortie couplée au générateur, ce procédé comprenant les étapes consistant à :
placer l'instrument de traitement dans le logement ;
déclencher l'alimentation en gaz de l'instrument et déclencher simultanément le transfert d'une salve d'énergie r.f. à l'instrument, ayant une forme d'onde et une durée prédéterminées de façon à générer un jet de plasma qui sort de la buse et heurte l'élément cible ;
évaluer automatiquement le signal de calibrage généré par le dispositif de calibrage à ladite sortie et représentatif d'une sortie du transducteur résultant du chauffage de l'élément cible par le jet de plasma ; et
régler le niveau d'énergie r.f. en sortie du générateur selon l'évaluation faite du signal de calibrage.
